# EUROPEAN PATENT SPECIFICATION

(11) **EP 3 442 497 B1**
(45) Date of publication and mention of the grant of the patent: **09.06.2021**
(21) Application number: 16753923.8
(22) Date of filing: 19.08.2016
(51) Int. Cl.: A61K 8/64, A61Q 7/00, A61K 8/9789

(54) **ACTIVE COMBINATIONS, COMPOSITIONS AND METHODS FOR ENHANCING HAIR GROWTH**
WIRKSTOFFKOMBINATIONEN, ZUSAMMENSETZUNGEN UND METHODEN ZUR FÖRDERUNG DES HAARWACHSTUMS
COMBINAISONS D'ACTIFS, COMPOSITIONS ET MÉTHODES POUR L'AMÉLIORATION DE LA POUSSE DES CHEVEUX

(30) Priority: 21.08.2015 US 201562208012 P
(43) Date of publication of application: 20.02.2019
(73) Proprietor: Gratia Mundi GmbH, 44795 Bochum (DE)
(72) Inventor: CABELLO ESCRIBANO, José Miguel, 65510 Idstein (DE)
(74) Representative: Fuchs Patentanwälte Partnerschaft mbB
(86) International application number: PCT/EP2016/069705
(87) International publication number: WO 2017/032711

(56) References cited:
- EP-A2- 2 561 858
- WO-A1-2014/120793
- FR-A1- 2 857 597
- US-A1- 2011 052 568
- DATABASE GNPD [Online] MINTEL; 1 April 2015 (2015-04-01), "Toatal Lash Serum", XP002761822, Database accession no. 3095983
- DATABASE GNPD [Online] MINTEL; 1 January 2015 (2015-01-01), "Pure Free Eyebrow Colour", XP002761823, Database accession no. 2941853
- DATABASE GNPD [Online] MINTEL; 1 February 2014 (2014-02-01), "Hair Growth Serum", XP002761824, Database accession no. 2280895
- LucasMeyer Cosmetics: "Capixyl(TM) Anti-Aging Hair Care Complex", , 5 April 2013 (2013-04-05), XP002761825, Retrieved from the Internet: URL:http://www.hatumou-life.com/wp-content /uploads/capixyl_kanzen.pdf [retrieved on 2016-09-14]

## Description

### Field of the invention

The present invention relates to active combinations and compositions for promoting growth of hair, in particular eyelashes and/or eyebrows, their use and cosmetic methods utilizing the composition of this invention.

The active combinations of this invention relate to synergistic mixtures of active ingredients, including e.g. botanical extracts and peptides. The active combinations are suitable to enhance hair growth in general. Surprisingly, the combinations even enhance hair growth in animals, e.g. horses, dogs, cats and rabbits. Preferred embodiments of this invention relate to compositions that include the active combinations of this invention. The compositions can be used to enhance hair growth. Specific combinations disclosed herein are particularly useful to enhance growth of the eyelashes and eyebrows in humans.

The compositions of this invention are preferably vegan, i.e. no animal products have been used, and no animal products have been used in the production of the ingredients of the compositions of this invention.

### Background of the invention

Compositions and methods for enhancing the growth of hair are known in the art. However, most methods are ineffective, others have severe side effects.

WO 2013/039597 A2 relates to a method for promoting hair growth. The compositions used in that method contain highly active pharmaceutical ingredients like 5-alpha reductase inhibitors (e.g. finasteride, dutasteride, progesterone) or prostaglandin analogs. Such active ingredients may cause severe side effects like impotence, anxiety, depression. These side effects are inacceptable in a cosmetic composition.

CA 2 735 825 teaches a hair growth promoting agent comprising 15,15-difluoroprostaglandin F2a derivatives. This compound is not only a highly active pharmaceutical compound that comes with a plethora of side effects but is also very lipophilic making it difficult to formulate in an aqueous composition. Aqueous compositions are generally relatively easily formulated, environmentally friendly and harmless; importantly, most subjects would not accept an oily, i.e. non-aqueous composition, as it would impart a very glossy appearance to the hair and/or skin. The modes of administration include oral administration, which is not desired as it extends the side-effects of a composition to systemic side effects. Local administration is always preferred. The same considerations apply to EP 1 558 203 B1, which describes compositions comprising styryl-pyrazole compounds.

US 8,197,865 B2 discloses compositions for modulating hair growth and regrowth. The compositions may contain extracts from a multitude of herbs, including red clover. The specific part of the description relates to extracts of green rooibos, saw palmetto and shiso extracts. The compositions contain lichochalcone. There is no disclosure of specific extracts. It is mentioned that red clover leaves can be used as an example. However, the example section does not mention any specific part of the plant used for preparation of the extracts. Further, the extraction liquid is DMSO, which is toxic to kidneys and liver.

WO 2014/120793 A1 teaches a hair loss treatment composition comprising hinoki oil, red clover extract and a peptide. Hinoki oil is oil made from a specific Japanese cypress tree. A red clover extract from the flower using ethanol as the solvent is recommended. Volunteers tested the product and reported reduction of hair loss after 4 to 6 weeks of using the product. Consumers usually do not continue using a product for such a long period before realizing any effect.

There is also known the "Capixyl(TM) Anti-Aging Hair Care Complex" (information retrievable from the Internet under URL http://www.hatumou-life.com/wp-content/uploads/ capixyl_kanzen.pdf). The Capixyl(TM) active product promotes hair growth and in particular also length, number and density of eyelashes. Capixyl(TM) comprises a mixture of acetyl tetrapeptide-3 and Trifolium pratense (clover) flower extract. Intense treatment is with a 5 wt.-% active containing composition.

In many hair growth compositions of the prior art alcohol based vehicles are used for administering the active ingredient. Alcohols like ethanol however make the hair brittle and leave a very cold feeling on the skin. Alcohols may also cause skin irritation.

Further, many compositions of the prior art are not suitable for vegans. Vegans chose not to consume any product that contains animal derived compounds. A distinction can be made between dietary and ethical vegans. Dietary vegans do not eat any animal products, whereas ethical vegans oppose the use of animals for any purpose. It is often surprising which ingredients of food and cosmetic products have been produced using animal derived products. For example, many polysaccharides are produced using lactose fermentation with whey as a raw material in the productions process. Needless to say that lactose is not suitable for vegans but used in many cosmetics and food. The same applies to many other ingredients to food and cosmetics.

Therefore, it would be useful to have an active combination, a cosmetic composition and a method that enhances hair growth, in particular of the eyebrows and eyelashes without containing any animal derived products or products that have been produced using animal-derived products as a raw material or additive. The combinations, compositions and methods of this invention should also be acceptable under the rules of ethical veganism, i.e. they should not contain any animal derived products, or products that have been prepared by the use of animals or animal products. Also, the combinations and compositions should be easily formulated, environmentally friendly and harmless. Further, it would be good if the compositions were free of highly active pharmaceutical ingredients like 5-alpha reductase inhibitors (e.g. finasteride, dutasteride, progesterone) or prostaglandin analogs. They should also be easy to use and preferably applied locally. Finally and most importantly, the combinations, compositions and methods should be effective in enhancing hair growth, in particular eyebrow and eyelash growth. It would be good to have a product that exerts its positive effects more quickly than the compositions of the prior art, preferably after 2 to 3 weeks.

### Description of the invention

The present invention relates to active combinations and cosmetic compositions comprising
- a botanical extract of *Vigna radiata,*
- a botanical extract of the flower of Trifolium pratense, and
- a peptide comprising the amino acid sequence Gly-His-Lys from N-terminal to C-terminal end.

The *V. radiata* extract is an extract of the sprout of the mung bean. It has surprisingly been found that a combination of the two ingredients leads to improved hair growth enhancing properties in the compositions and active combinations.

The inventors believe that the magnificent activity of the inventive active combinations and compositions is at least partly based on the specific combination of the extract of mung bean sprouts (*Vigna radiata*) and the peptide. Mung bean sprouts contain isoflavones which are also referred to as phytoestro-gens. Examples of isoflavones that are preferably contained in the active combinations and compositions of this invention are biochanin A, formononetin, daidzein and genistein. The following structural formulae illustrate the isoflavones that are preferably present in the compositions and active combinations of this invention. 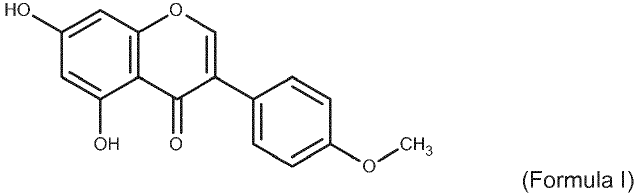 biochanin A: 5,7-dihydroxy-3-(4-methoxyphenyl)chromen-4-one 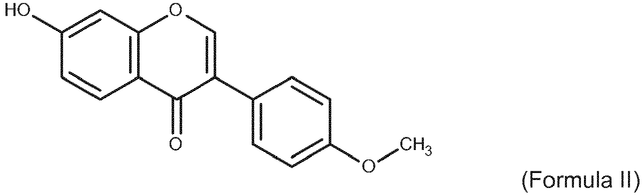 formononetin: 7-dihydroxy-3-(4-methoxyphenyl)chromen-4-one 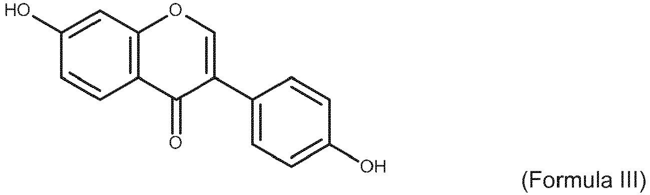 daidzein: 7-hydroxy-3-(4-hydroxyphenyl)chromen-4-one 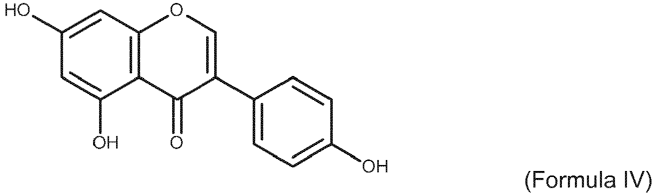 genistein: 5,7-dihydroxy-3-(4-hydroxyphenyl)chromen-4-one

It is not clear which of the isoflavones is responsible for the beneficial effects that the active combinations and compositions of this invention have on hair growth and in particular on eyebrow and eyelash growth. Probably, it is the combination of isoflavones in the extract or extracts used for this invention that leads to the beneficial effects. The specific glycosylation may have an influence on the activity as well. Every plant and even every part of a plant has its unique isoflavone fingerprint that contributes to the activity profile of the botanical extract made from that part of the plant. The inventors hypothesize that the amount of biochanin A in the compositions of this invention should be at least 100 ppb, more preferably at least 250 ppb, most preferably at least 400 ppb or even at least 500 ppb, or even more than 750 ppb (m/m). However, the amount of biochanin A should also not be too high in order to rule out any undesired side effects. Hence, the total amount of biochanin A should be limited to at most 5 ppm, preferably at most 3 ppm, more preferably at most 2 ppm. Such preferable amounts of biochanin A are achieved by the use of extracts of red clover, which are preferably contained in the active combinations and compositions of this invention. In preferred embodiments, the combinations and compositions of this invention comprise at least one, preferably all of the following compounds: biochanin A, formononetin, daidzein, daidzin, genistein, genistin, catechin, myricetin, myricetin-3-O-arabinoside, quercetin, quercetin-3-O-glucoside, quercetin-3,4'-O-diglusocide, baicalein, kaempferol, kaempferol-3-O-glucosyl-rhamnosyl-glucoside and/or kaempferol-7-O-glucoside. The combinations and compositions of this invention may also comprise one or more of the following compounds: p-coumaric acid ethyl ester, p-coumaric acid-4-O-glucoside, 3,7-dimethyl quercetin, apigenin-7-O-glucoside, apigenin-7-O-(6"-malonyl-apiosyl-glucoside, apigenin-7-O-apiosyl-glucoside, myricetin-3-O-galactoside, luteolin-7-O-glucosid, 6-prenylnaringenin, luteolin-7-O-(2-apiosyl-6-malonyl)-glucoside, luteolin-7-O-(2-apiosyl-glucoside) and/or luteolin-7-O-malonyl-glucoside.In preferred embodiments of this invention the active combinations and compositions comprise at least one additional botanical extract. The additional botanical extract is preferably selected form extracts of the sprouts of red clover, i.e. *Trifolium pratense.* In other words, in an embodiment the inventive active combinations and compositions comprise the extracts of *V*. *radiata* sprouts and *T. pratense* flowers; in another embodiment the active combinations and compositions comprise the extracts of *V*. *radiata* sprouts, *T. pratense* sprouts and *T. pratense* flowers.

It is preferred that the weight amount of *V*. *radiata* sprout extract in the active combinations and compositions of this invention exceeds the weight amount of *T. pratense* flower extract. In preferred embodiments the weight amount of *V. radiata* sprout extract in the active combinations and compositions of this invention exceeds the weight amount of *T. pratense* flower extract by a factor of at least 2, more preferably at least 3, more preferably at least 4, more preferably at least 5, more preferably at least 6 and most preferably at least 7, or even at least 10. It is preferable that the weight amount of *V*. *radiata* sprout extract in the active combinations and compositions of this invention exceeds the weight amount of *T. pratense* flower extract by a factor of not more than 30, more preferably not more than 20, more preferably not more than 15. In certain embodiments, the weight amount of *V*. *radiata* sprout extract in the active combinations and compositions of this invention exceeds the weight amount of *T. pratense* flower extract by a factor of not more than 12, more preferably not more than 11 and most preferably not more than 10.

It is preferred that the weight amount of *T. pratense* sprout extract in the active combinations and compositions of this invention exceeds the weight amount of *T. pratense* flower extract. In preferred embodiments the weight amount of *T. pratense* sprout extract in the active combinations and compositions of this invention exceeds the weight amount of *T. pratense* flower extract by a factor of at least 2, more preferably at least 3, more preferably at least 4, more preferably at least 5, more preferably at least 6 and most preferably at least 7, or even at least 10. It is preferable that the weight amount of *T*. *pratense* sprout extract in the active combinations and compositions of this invention exceeds the weight amount of *T. pratense* flower extract by a factor of not more than 30, more preferably not more than 20, more preferably not more than 15. In certain embodiments, the weight amount of *T. pratense* sprout extract in the active combinations and compositions of this invention exceeds the weight amount of *T. pratense* flower extract by a factor of not more than 12, more preferably not more than 11 and most preferably not more than 10.. When amounts, including relative amounts, of botanical extracts are indicated herein, these indications in particular relate to the weight amount of the dried extracts. All indications of amounts - unless otherwise mentioned - relate to amounts by weight.

The relative amounts of botanical extracts in the active combinations and compositions of this invention are chosen such that the isoflavone fingerprint is beneficial for enhancing hair growth, in particular eyebrow and eyelash growth.

It is preferred that the weight amount of *V*. *radiata* sprout extract in the active combinations and compositions of this invention exceeds the weight amount of the peptide. In preferred embodiments the weight amount of *V*. *radiata* sprout extract in the active combinations and compositions of this invention exceeds the weight amount of the peptide by a factor of at least 2, more preferably at least 3, more preferably at least 4, more preferably at least 5, more preferably at least 6 and most preferably at least 7, or even at least 10. It is preferable that the weight amount of *V*. *radiata* sprout extract in the active combinations and compositions of this invention exceeds the weight amount of the peptide by a factor of not more than 30, more preferably not more than 20, more preferably not more than 15. In certain embodiments, the weight amount of *V*. *radiata* sprout extract in the active combinations and compositions of this invention exceeds the weight amount of peptide by a factor of not more than 12, more preferably not more than 11 and most preferably not more than 10.

It is preferred that the weight amount of *T. pratense* sprout extract in the active combinations and compositions of this invention exceeds the weight amount of the peptide. In preferred embodiments the weight amount of *T*. *pratense* sprout extract in the active combinations and compositions of this invention exceeds the weight amount of the peptide by a factor of at least 2, more preferably at least 3, more preferably at least 4, more preferably at least 5, more preferably at least 6 and most preferably at least 7, or even at least 10. It is preferable that the weight amount of *T. pratense* sprout extract in the active combinations and compositions of this invention exceeds the weight amount of the peptide by a factor of not more than 30, more preferably not more than 20, more preferably not more than 15. In certain embodiments, the weight amount of *T. pratense* sprout extract in the active combinations and compositions of this invention exceeds the weight amount of peptide by a factor of not more than 12, more preferably not more than 11 and most preferably not more than 10.

The inventors believe that the concomitant use of the extract or extracts of this invention with the peptide described herein provides for a synergistic effect in the treatment of hair loss and in enhancing hair growth, in particular of the eyelashes and eyebrows.

The use of red clover extracts in compositions for enhancing hair growth is not as such novel, as can be seen from WO 2014/120793 A1. The focus in the art has always been on the flower extracts of red clover because the flower extract has a stronger estrogenic effect than the sprout extract, probably due to the higher amount of O-methylated isoflavones. Mung beans on the other hand were reported to have almost no estrogenic effect at all. The specific combination of botanical extracts of the present invention is much more effective than known compositions containing red clover extracts. The active combinations and compositions of the present invention have been clinically tested and it has been proven that positive results can be observed already after 15 days of using the active combinations and compositions. Particularly, the setup and outcome of said clinical study is described in Example 4 hereinafter. Briefly, the efficacy of the composition of the present invention in comparison to placebo treatment was assessed with a group of 15 female volunteers, who applied the composition and the placebo once a day for 60 days. The subjects applied the active composition of the present invention at the roots of lashes on one eye and the placebo on the second eye. The evaluations performed before and after 15, 30 and 60 days showed a statistically significant increase in the eyelashes maximum length after 30 and 60 days of application of the composition of the present invention, which reflects an increase of +8.1% at the end of the test. Moreover, a statistically significant increase in the eyelashes average length after 30 and 60 days of application of the composition of the present invention of +10.3% at the end of the test could be observed. Furthermore, a statistically significant increase in the number of eyelashes after 30 and 60 days of application of the composition of the present invention of +11.9% at the end of the test could be observed. By contrast, no significant variation was found for the placebo treatment. Still further, after 15, 30 and 60 days statistically significant difference between the application of the composition of the present invention and the placebo treatment were evidenced. Thus, it has been shown that the combinations and compositions of this invention show very good results even after short-term treatment.

In preferred embodiments the active combinations and compositions of this invention comprise the extracts of *V*. *radiata* sprouts and *T. pratense* sprouts as well as *T. pratense* flowers, wherein the weight amounts of each of the two sprout extracts individually exceed the amount of the flower extract and/or the amount of the peptide.

The peptide that is used in the active combinations and compositions of this invention comprises the amino acid sequence Gly-His-Lys from N-terminal to C-terminal end. Preferably, the peptide is N-acetylated.

Nonlimiting examples of peptides suitable for use in the active combinations and compositions include tripeptide-1, acetyl hexapeptide-8, acetyl dipeptide-1, caproyl tetrapeptide-3, carnosine, glutathione, marine oligopeptide, palmitoyl oligopeptide, human oligopeptide-1 (EGF), acetyl tetrapeptide-3, palmitoyl tetrapeptide-7, acetyl tetrapeptide-5, palmitoyl hexapeptide-14, pentapeptide-3, nonapeptide-1, acetyl hexapeptide, hexapeptide-11, SH-polypeptide-15, hexanoyl dipeptide-3, acetyl octapeptide-3, palmitoyl tripeptide-5, palmitoyl dipeptide-5, palmitoyl dipeptide-6, acetyl tetrapeptide-2, and myristoyl pentapeptide-17, a peptide compound characterized by formula X and/or a peptide conjugate compound characterized by formula XI, or combinations thereof:

Z-Gly-His-Lys (formula X)

A-Z-Gly-His-Lys (formula XI)

wherein A represents the radical corresponding to a monocarboxylic acid of general formula R-COOH, where R represents a linear or branched C₁-C₂₄ aliphatic radical optionally substituted with a hydroxyl group. In an embodiment, such monocarboxylic acid is unsaturated. In an embodiment, the monocarboxylic acid comprises lipoic acid or its reduced form, dihydrolipoic acid, N-lipoyl lysine, retinoic acid, or combinations thereof. Z represents 1 to 3 Lys residues that are optionally methylated. In the case of formula XI, Z may represent a covalent bond. The monocarboxylic acid A is preferably acetic acid which is linked to a lysine residue forming a carboxylic acid amide linkage.

In preferred embodiments, the peptide is present in the combinations and compositions of this invention in an amount of more than 1 ppm, preferably more than 2 ppm, more preferably at least 5 ppm, most preferably at least 8 ppm. In an embodiment, the amount of peptide is limited to a maximum of 50 ppm, more preferably a maximum of 30 ppm and most preferably a maximum of 20 ppm. The peptide is included in the active combinations and compositions in order to stimulate extracellular matrix proteins for stronger hair anchoring.

The peptides of this invention have shown to exert a synergistic effect when used together with the botanical extracts of the active combinations and compositions of this invention.

### Compositional aspects

The present invention also relates to a cosmetic composition for enhancing growth of the eyelashes and/or eyebrows in a subject, the composition comprising at least one botanical active ingredient, a hair growth promoting peptide, water and a viscosity adjusting agent, wherein the composition is vegan. In preferred embodiments the composition comprises the active combination of this invention. The composition has been clinically tested and showed an overwhelming effect.

The composition of this invention is preferably liquid. The composition preferably contains water. The amount of water in the composition is preferably selected to be at least 50% by weight, in particular from 50 to 98% by weight. Preferred ranges include 55 to 97% by weight, 65 to 95% by weight and 75 to 90% by weight. Water is acceptable in the vegan diet. Preferably, water is the main ingredient of the compositions, i.e. it exceeds the amounts of all other ingredients in the composition on a weight basis.

In preferred embodiments the viscosity of the composition is at least 0.8 mPas, more preferably at least 1 mPa, more preferably at least 2 mPa, more preferably at least 5 mPa and most preferably at least 10 mPa. Preferred compositions have viscosities of not more than 10⁴ mPas, preferably not more than 10³ mPas and most preferably not more than 10² mPas. Preferably, the viscosity is determined using a flow cup viscometer, preferably in accordance with the method described in ASTM D 1200:1994 and/or DIN EN ISO 2431:2011. The viscosity is preferably determined at 25°C. It is necessary to choose the right viscosity for the compositions of this invention so that the compositions can be applied conveniently. Further, the compositions are preferably so-called leave-on compositions that should remain on the part of skin or hair where the user has applied them. Therefore, the viscosity should not be too low or too high. The compositions are particularly useful for enhancing eyebrow and eyelash growth. The viscosity and overall properties have been adjusted for that purpose.

Viscosity can be adjusted using viscosity adjusting agents. Preferably, the compositions comprise one or more viscosity adjusting agents in a cumulative amount of at least 0.01% by weight, more preferably at least 0.05% by weight, more preferably at least 0.1% by weight and most preferably at least 0.15% by weight of the composition. In preferred embodiments, the amount of viscosity adjusting agents does not exceed 15% by weight, more preferably the maximum amount is ≤10% by weight, more preferably ≤5% by weight, more preferably ≤1% by weight and most preferably ≤0.5% by weight.

The viscosity adjusting agents are preferably selected from the polysaccharides. "Polysaccharides" in the context of this invention includes saccharides polymers and oligomers having at least 3 monosaccharide moieties.

Preferably, the viscosity adjusting agents are selected from the group consisting of gellan gum, xanthan gum, dextran and mixtures thereof. In preferred embodiments, the compositions of this invention comprise gellan gum, xanthan gum and dextran. Preferably, the amount of xanthan gum and/or gellan gum exceeds the amount of dextran on a weight basis.

Preferred embodiments of the inventive compositions contain xanthan gum in an amount of from 0.01 to 1% by weight, preferably from 0.05 to 0.5% by weight and most preferable from 0.08 to 0.15% by weight. Xanthan gum is conventionally prepared using egg white (e.g. Keltrol® RD sold by Rahn GmbH), which is not acceptable in a composition for ethical vegans. Therefore, preferred embodiments contain xanthan gum that has been produced without the use of egg white. An example of such a preferred xanthan gum is Keltrol® CG-RD from Rahn GmbH.

Preferred embodiments of the inventive compositions contain gellan gum in an amount of from 0.01 to 1% by weight, preferably from 0.05 to 0.5% by weight and most preferable from 0.08 to 0.15% by weight. An example of a preferred gellan gum is Kelcogel® CG-HA from Rahn GmbH.

Preferred compositions of this invention comprise dextran in an amount of less than 1% by weight, preferably from 0.001 to 0.1% by weight, more preferably from 0.002 to 0.05% by weight.

The composition of this invention is preferably colorless. This allows application of the active combination and/or composition of this invention to visible body parts, particularly the subject's eye lids and/or eyebrows, without changing of appearance of the subject in a disturbing way. Accordingly, customer compliance to application is increased.

The compositions of this invention preferably comprise at least one preservative with skin-conditioning properties or a mixture of preservatives with skin-conditioning properties, in particular in an amount of from 2 to 20% by weight. Using preservatives with skin-conditioning properties has the benefit that two effects can be achieved, i.e. a preserving effect on the composition and a moisturizing effect on the subject's skin. If the amount of these preservatives with skin-conditioning properties is too high, viscosity of the compositions will increase. If the amount of preservatives with skin-conditioning properties in the compositions is too low, the preservative effect and also the skin-conditioning properties will not be achieved. For this reason preferred compositions include preservatives with skin-conditioning properties in an amount of from 3% by weight to 15% by weight, preferably from 6% by weight to 13% by weight and more preferably at least 8% by weight of the composition.

Preferred preservatives with skin-conditioning properties are polyhydric alcohols. Preferred polyhydric alcohols are alcohols having from two to five, preferably two or three hydroxyl groups. Preferred compounds are glycols. Preferred preservative compounds with skin-conditioning properties include pentylene glycol (e.g. 1,2-pentanediol), propanediol (e.g. 1,2-propanediol), butylene glycol (e.g. 1,3-butanediol), glycerol and mixtures thereof.

In a preferred embodiment, the compositions of this invention comprise the following ingredients:

**Table 1**

| **ingredient** | **minimum (by weight)** | **maximum (by weight)** |
|---|---|---|
| water | 50% | 98% |
| preservative with skin-conditioning properties | 2% | 20% |
| viscosity adjusting agents | 0.01% | 15% |
| low dose preservative | 0.001% | 0.01% |
| botanical extracts | 0.001% | 0.1% |
| peptide | 0.0001% | 0.01% |

The low dose preservatives are preferably selected from preservatives that are active at concentrations of less than 1% by weight. Preferred compounds that have this property are sorbates and benzoates, e.g. sodium benzoate and potassium sorbate.

In preferred embodiments the compositions of this invention essentially consist of the ingredients listed in table 1. In alternative embodiments the compositions of this invention essentially consist of the ingredients mentioned in table 1 to an extent of at least 95% by weight, more preferably at least 98% by weight, most preferably at least 99% by weight.

In preferred embodiments the compositions of this invention are free of highly active pharmaceutical ingredients. "Free of" in the context of this invention means that the compounds are not added to the compositions on purpose. If contained at all, such compounds will be contained in an amount of less than 100 ppm, more preferably less than 10 ppm, more preferably less than 1 ppm and most preferably less than 10 ppb.

In a preferred embodiment the compositions of this invention are free of parabens. Parabens have been reported to cause skin irritation and allergies. Hence, these substances are preferably not present in the compositions.

All indications of amounts in this description relate to amounts by weight unless otherwise mentioned.

The pH of the compositions of this invention is preferably in a range of less than 7, in particular from 5 to 6. A slightly acidic pH is preferred because the activity of the low dose preservatives is enhanced under acidic conditions.

In particularly preferred embodiments of this invention the compositions of this invention are free of animal products and preferably also free of products that have been made using animal products. Ethical veganism has increasing followers and many customers oppose the use of animals for any purpose. Therefore, it is one aspect of this invention that the compositions can be made without and do not contain any animal products and/or products obtained using animals.

**Table 2**

| **ingredient** | **minimum (by weight)** | **maximum (by weight)** |
|---|---|---|
| carrier | | |
| water | 50% | 98% |

| preservatives with skin-conditioning properties | | |
|---|---|---|
| pentylene glycol | 3% | 7% |
| butylene glycol | 0.5% | 2.5% |
| glycerol | 0.2% | 5%, preferably 2% |
| propanediol | 2.5% | 6% |

| viscosity adjusting agents | | |
|---|---|---|
| xanthan gum | 0.01% | 1% |
| gellan gum | 0.01% | 1% |
| dextran | 0.001% | 0.1% |

| **ingredient** | **minimum (by weight)** | **maximum (by weight)** |
|---|---|---|
| low dose preservatives | | |
| sodium benzoate | 0.001% | 0.015% |
| potassium sorbate | 0.001% | 0.01% |

| botanical extracts | | |
|---|---|---|
| T. pratense flower | 0.0001% | 0.01% |
| T. pratense sprout | 0.001% | 0.1% |
| V. radiata sprout | 0.001% | 0.1% |

| other active ingredients | | |
|---|---|---|
| peptide | 0.0001% | 0.01% |

In preferred embodiments the compositions of this invention consist of the ingredients listed in table 2. In alternative embodiments the compositions of this invention consist of the ingredients mentioned in table 2 to an extent of at least 95% by weight, more preferably at least 98% by weight, most preferably at least 99% by weight.

In preferred embodiments the compositions of this invention comprise the ingredients in the following list in descending order based on the weight amount present in the composition:
water > preservatives with skin-conditioning properties > viscosity adjusting agents > botanical extracts > low dose preservatives > peptides.

In preferred embodiments the compositions of this invention comprise the ingredients in the following list in descending order based on the weight amount present in the composition:
water > pentylene glycol > propanediol > butylene glycol > glycerol > viscosity adjusting agents > sprout extracts > low dose preservatives > flower extracts.

With regard to the active ingredients in the composition it is preferred that the active combinations and compositions of this invention comprise the active ingredients in accordance with at least one - preferably all - of the following criteria A to D (based on the weight amount of dry extract) in the active combinations and compositions:
A. sprout extracts > flower extracts
B. *T. pratense* extracts > *V. radiata* extract
C. *T. pratense* sprout extract > *T. pratense* flower extract
D. *V. radiata* extract > *T. pratense* flower extract

The relationship between the extracts used in the active combinations and compositions determines the amount of active botanical ingredients in the active combinations and compositions of this invention. In preferred embodiments at least one - preferably all - of the following criteria E to G are met (based on the weight amount of dry extract) in the active combinations and compositions:
E. sprout extracts > flower extracts x 10
F. *T. pratense* sprout extract > *T. pratense* flower extract x 5
G. *V. radiata* extract > *T. pratense* flower extract x 5

The active combinations and compositions of this invention preferably comprise active botanical ingredients. The active combinations and compositions of this invention advantageously contain biochanin A (Formula I) and formononetin (Formula II), i.e. the O-methylated isoflavones. However, unlike most hair growth enhancing agents of the prior art the present active combinations and compositions are not designed to maximize the amount of O-methylated isoflavones. The inventors believe that the O-unmethylated isoflavones could be the decisive compounds in the active combinations and compositions. O-unmethylated isoflavones that are preferably contained in the active combinations and compositions of this invention by virtue of the choice of botanical extracts, in particular the *V. radiata* sprout extract, are daidzein (Formula III) and genistein (Formula IV). O-unmethylated compounds have antioxidant properties by scavenging reactive oxygen species. Other O-unmethylated compounds that are preferably contained in the active combinations and compositions of this invention are catechin (Formula V), myricetin (Formula VI), quercetin (Formula VII), baicalein (Formula VIII), kaempferol (Formula IX) or mixtures thereof.

Additionally or alternatively the active combinations and compositions of this invention may contain glycosides of the isoflavones mentioned herein. Preferred glycosides are selected from the group of the glucosides, arabinosides, galactosides, glucoronides and mixtures thereof. 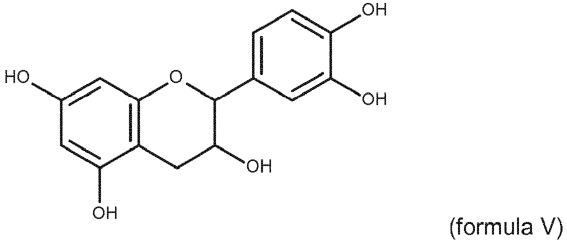 catechin: 2-(3,4-dihydroxyphenyl)-3,4-dihydro-2H-chromene-3,5,7-triol 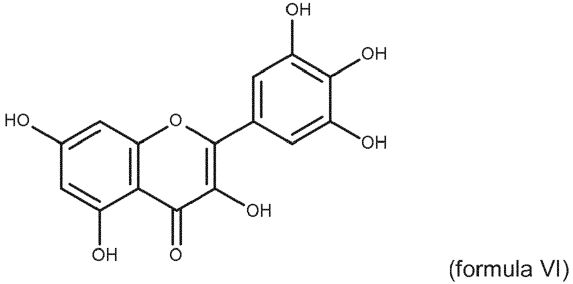 myricetin: 3,5,7-Trihydroxy-2-(3,4,5-trihydroxyphenyl)-4-chromenone 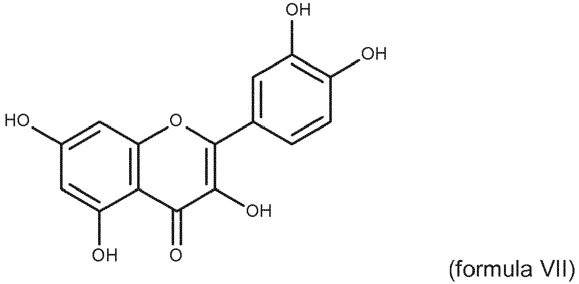 quercetin: 2-(3,4-dihydroxyphenyl)-3,5,7-trihydroxy-4H-chromen-4-one 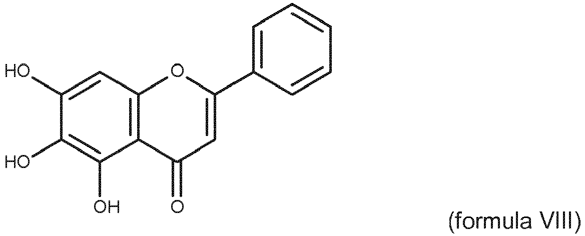 baicalein: 5,6,7-Trihydroxy-2-phenyl-chromen-4-one 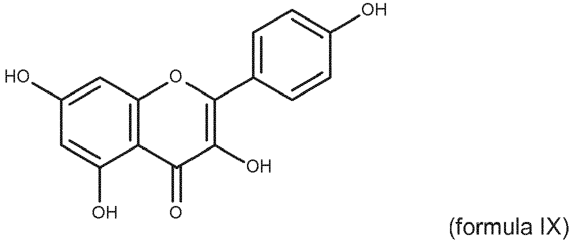 kaempferol: 3,5,7-Trihydroxy-2-(4-hydroxyphenyl)-4H-chromen-4-one

It has surprisingly been found that the compositional details described herein allow for formulation of cosmetic compositions that do not contain any animal derived products or products that have been prepared using animal products.

The compositions of this invention are preferably not-irritant or at least hypoirritant. The irritation properties of the composition of this invention can be advantageously assessed by determining its S-value. The S-value can be preferably assessed by an Irritation Test as described in Example 3 herein. The composition of this invention preferably has an S-value of less than 6, more preferably of less than 5, still more preferably of less than 4 and most preferably of 3 or less.

### Preparation of extracts

In order to achieve the desired combination of active botanical ingredients it is preferred that the extracts used in the active combinations and compositions of this invention are prepared such that the amount of O-unmethylated isoflavones is increased. O-unmethylated isoflavones are more hydrophilic than O-methylated isoflavones because a hydroxyl group imparts the molecule with improved water solubility when compared to the methyl ether group of the O-methylated compounds. Hence, it is preferred that a hydrophilic solvent is used for the extraction of the plants or plant parts used in this invention.

Preferably, the extracts of the sprouts are prepared by sprouting the seeds of *T. pratense* and *V. radiata,* respectively. Sprouting is preferably initiated by wetting the seeds with water and keeping them in a light and warm place. After sprouting of the seeds, the sprouts obtained will be subjected to extraction with at least one extraction liquid. The extraction liquid can be an organic solvent, water or a mixture of one or more organic solvents and water. Organic solvents include acetic acid, diethyl ether, ethyl acetate, alcohols (e.g. methanol, ethanol, isopropanol, butanol), dichloromethane, chloroform, hexane, benzene, toluene, xylene, petroleum ether and combinations thereof. In preferred embodiments the extraction liquid is selected from alcohols, water and mixtures thereof. Hydrophilic extraction liquids are preferred. The pH of the extraction liquid may be acidic, neutral or alkaline.

Preferably, the extracts of the flowers are prepared by subjecting the flowers to extraction with at least one extraction liquid. The extraction liquid can be an organic solvent, water or a mixture of at least one organic solvent and water. Organic solvents include acetic acid, diethyl ether, ethyl acetate, alcohols (e.g. methanol, ethanol, isopropanol, butanol), dichloromethane, chloroform, hexane, benzene, toluene, xylene, petroleum ether and combinations thereof. In preferred embodiments the extraction liquid is selected from alcohols, water and mixtures thereof. Hydrophilic extraction liquids are preferred. The pH of the extraction liquid may be acidic, neutral or alkaline.

The collected extract is preferably filtered to remove undesired plant components. The extract may be concentrated, in particular by solvent removal, e.g. distillation. In preferred embodiments, the extract is lyophilized. Using lyophilized extracts has the advantage that solubility in water of the extract is enhanced, which is beneficial for the active combinations and compositions of this invention. Preferably, at least one of the extracts is a dry extract. Preferably, all of the extracts are dry extracts before being used in the compositions of this invention. If nothing else is indicated in this description, all weight amounts of extracts relate to the respective dry extract, in particular lyophilized extract, i.e. substantially not containing any solvents.

Each plant has a number of structural parts. Extracts can usually be made from every part, like leaves, flowers, roots, sprouts, fruit, wood, bark, stems etc. The different parts have different amounts of the plant's ingredients. Some parts may be free of substances that are included in high amounts in other parts. A good example is the potato plant that contains nutritious starch in its tubers but has toxic solanine in its leaves and fruit. In the compositions of the present invention the extracts of *Trifolium pratense* are preferably prepared from the flower and/or sprouts of the red clover plant. With regard to the mung bean it is preferred that the sprouts are used for preparation of the extract. In particular the sprouts of red clover and mung beans have shown to contain considerable amounts of O-unmethylated isoflavones, leaving a desirable isoflavone fingerprint in the products of this invention. The extracts used in this invention have been proven to be safe to use.

### Cosmetic method

The present invention also relates to a cosmetic method for enhancing hair growth in a subject, the method comprising the steps of
- administering to the subject an effective amount of an active combination and/or composition in accordance with this invention.

In preferred embodiments the subject is a human. The human can be male or female. In most embodiments the human is a female. In a preferred embodiment the human is a vegan, in particular an ethical vegan.

In alternative embodiments the compositions of this invention can be applied to animals. Preferably, these animals include dogs, horses, rabbits and cats. The compositions can be applied to other animals as well.

The active combination and/or composition of this invention is preferably administered to the subject by application of the active combination and/or composition to the site of the subject at which the subject is suffering from decreased hair density and/or decreased hair length and/or hair loss. Particularly, the active combination and/or composition can be applied to a subject's eyelids and/or roots of eyelashes and/or eyebrows. However, in case the subject is an animal, particularly sites of fur loss and/or less dense fur can be used as site of application. In a particularly preferred embodiment the site of application is the root of mane or tail.

In preferred embodiments the active combination and/or composition is administered to the subject at least once a day. Preferred embodiments relate to the administration of the active combination and/or composition to a subject at least once a day, preferably twice a day, for a period of at least 3, more preferable at least 5, more preferably at least 10, at least 15, at least 20, at least 30, at least 40, at least 50 and most preferable at least 60 consecutive days. Experiments have shown that the active combination and/or composition show very remarkable results, in particular in terms of eyelash growth already after an application of 15 days. Said remarkable results can even be increased, in particular in terms of eyelash density, if the treatment with the active combination and/or composition of the invention is prolonged to a period of at least 30, more preferable at least 60 consecutive days. In a preferred embodiment the administration of the active combination and/or composition to a subject at least once a day is applied in the evening, preferably after 9 p.m. This may be of particular advantage for the efficacy of the treatment due to circadian rhythm and/or application comfort of the subject. However, in alternative embodiments, the combinations and/or compositions of this invention are applied twice a day or even three times a day.

In preferred embodiments of this invention the active combination and/or composition is administered to the subject by the subject itself or by a professional, e.g. a hairdresser, a cosmetician, a makeup artist, or a medical practitioner.

In preferred embodiments the active combination and/or composition will be applied to the subject's skin or hair on a part of the body in need of hair growth enhancing treatment. This part of the body can be selected from the body and the head, including the scalp, the eyebrows, the eyelashes, the eyelids, the periorbital part of the face, the perioral part of the face, the cheeks. Exemplary parts of the body that can preferably be treated are the breast, the arms, and the legs.

### Examples

### Example 1 - Preparation

The following table 3 shows an exemplary preferred nonlimiting composition of this invention that contains an inventive active combination. The composition was prepared by mixing the carrier with pentylene glycol, propanediol, xanthan gum and gellan gum. The obtained mixture was heated to 70°C and homogenized. Thereafter, the mixture was cooled while stirring and the remaining ingredients, including the active combination, added at temperatures of less than 30°C. The resulting composition is fluid and colorless.

**Table 3**

| **ingredient** | **amount (by weight)** | **purpose** |
|---|---|---|
| water | 87.771% | carrier |
| pentylene glycol | 2.000% | preservatives with skin-conditioning properties |
| butylene glycol | 2.500% | |
| glycerol | 1.400% | |
| propanediol | 6.000% | |
| xanthan gum | 0.200% | viscosity adjusting agents |
| gellan gum | 0.050% | |
| dextran | 0.045% | |
| sodium benzoate | 0.007% | low dose preservatives |
| potassium sorbate | 0.002% | |
| T. pratense flower | 0.004% | botanical extracts |
| T. pratense sprout | 0.006% | |
| V. radiata sprout | 0.012% | |
| acetyl tetrapeptide-3 | 0.003% | peptide |

### Example 2

The following table shows another preferably composition of this invention.

**Table 4**

| **ingredient** | **FDA amount code** | **purpose** |
|---|---|---|
| water | A | carrier |
| pentylene glycol | D | preservatives with skin-conditioning properties |
| propanediol | E | |
| glycerol | E | |
| butylene glycol | E | |
| dextran | F | viscosity adjusting agents |
| gellan gum | F | |
| xanthan gum | F | |
| T. pratense flower | G | botanical extracts |
| T. pratense sprout | G | |
| V. radiata sprout | G | |
| acetyl tetrapeptide-3 | G | peptide |
| sodium benzoate | G | low dose preservatives |
| potassium sorbate | G | |

| | | |
|---|---|---|
| FDA amount code: A:>50%, B:25-50%, C:10-25%, D:5-10%, E:1-5%, F:0.1-1%, G:<0.1% | | |

### Example 3 - Irritation Test

The composition prepared in Example 1 was subjected to a Hen's egg chorioallantoic membrane test for irritation ("HET-CAM test") as essentially described in Luepke (Luepke NP Hen's egg chorioallantoic membrane test for irritation potential - Chem. Toxic. 1985: 23 (2): 287-291) to assess irritation properties of the composition.

In connection therewith, a person skilled in the art will immediately acknowledge that said irritation test is a well-known method, which is acknowledged in the art for assessment of the irritation potential of a test sample. Thereby the appearance of irritative reactions on the chorioallantoic membrane of fertilized chicken eggs, as a response to the exposition of the membrane to the test sample is analyzed. The potential irritancy of a substance is assessed by observing the adverse reactions which occur in the chorioallantoic membrane of a fertile hen's egg after exposure to the tested substance. The chorioallantoic membrane (CAM) of fertilized chicken eggs is a highly vascularized structure inside the egg. Its exposure allows the direct observation of blood vessels. It is possible to apply a test sample onto the CAM surface and to observe the onset of hemorrhage, lysis and coagulation occurring on the vascular system and the albumin. The severity of the vascular damage of the chorioallantoic membrane therefore provides indications about the irritation potential of a test sample.

For this purpose, commercially available fertilized white chicken eggs without micoplasms were provided. Eggs were used on the 9^{th} day of incubation, after having been controlled for embryo viability. The eggs were opened near the air cell using a pair of surgical scissors. The section of the shell was carefully pared off to reveal the highly vascularized chorioallantoic membrane (CAM).

Test samples were prepared by suspension of the composition prepared in Example 1 in physiological buffer in concentrations of 1:2 and 1:3, respectively. As a further sample a part of the composition prepared in Example 1 was left undiluted.

Subsequently, 0.3 ml of either the prepared undiluted sample or the 1:2 or 1:3 diluted samples, respectively, were separately applied on the CAM surface of a series of 6 individual eggs for 300 seconds.

Subsequently, the sample was removed and the intensity of hemorrhage, lysis and coagulation occurring on the vascular system and the albumin was assessed. Thereby an individual score was given to each egg on the basis of the observed intensity of the irritant reactions categorized as either" + slightly visible reaction", "++ clearly visible reaction", or "+++ severe reaction".

The S-value, which represents the sum of the individual scores given to each egg on the basis of the intensity of the irritant reactions, was calculated. Thereby, the irritation potential of the test sample was "hypoirritant" if the S-value is less than 6, "slightly irritant" if the S-value is less than 12 but more than 6, "irritant" if the S-value is more than 12 but less than 16, and "strongly irritant" if the S-value is more than 16.

According to said procedure, the test samples of the composition prepared in Example 1 showed an S-value of 3 and were thus considered to be hypoirritant.

### Example 4 - Efficacy

For determining the efficacy of the composition of the present invention, the composition as shown in table 3 of Example 1 ("composition" in the following) was tested in a clinical study, wherein volunteers applied the composition to their eyelids. For this purpose, an efficacy test as described hereinafter was carried out according to the Declaration of Helsinki on 15 female human volunteering subjects having an average age of 47.9 years. The composition was thereby compared to a placebo application, whereby a comparative composition not containing the active combination was used as the placebo ("placebo" in the following).

The present inventors surprisingly found that the active treatment with the composition showed a statistical significant increase of the following parameters:
- maximum eyelashes length,
- average eyelashes length, and
- eyelashes density (number of eyelashes in a small area).

The efficacy of the active treatment with the composition is also confirmed as the results obtained in the area treated with the composition are also significantly different from those obtained in the area treated with the placebo.

### Subject selection

The subjects selected for participation in the study were of Caucasian race, female humans having an age of 18 - 70 years, having in general good health, being able to follow all study directions and to commit to all follow-up visits for the duration of the study, completing the informed consent process, and were avoiding the exposure to UV radiation and the use of tanning beds for the duration of the study.

The subjects selected for participation in the study were particularly not pregnant or nursing females, did not have a history of unusual skin reactions to skin care toiletry products, cosmetics, or sensitivity to any of the test article components, were not taking topical or systemic drugs that could affect the results of the test (anti-inflammatory agents, corticosteroids, etc), did not show systemic diseases or skin disorders (such as eczema, psoriasis, severe acne, etc.) that may affect the evaluation of the test articles or increase risk to the subject, were not currently using lengthening treatments for eyelashes, or were not currently involved in another clinical investigation or had been involved within a period of 30 days prior to admission in this study.

The 15 subjects were recruited for the study and did not wash their face for two hours before the parameter measurement and did not apply any product for 12 hours before the parameter measurement.

### Drop-out and Restrictions

The subject's participation in the study (*Drop-out*) was interrupted at free choice of the subject, for medical reasons not correlated with the treatment (e.g., onset of disease, surgical operation), or reasons correlated with the treatment (e.g., irritant or allergic reactions).

During the study, the subjects were instructed to wash their face using their current skin care regimen and not to apply the tested product on any other site than the prescribed ones. For the whole duration of the test, the subjects should not use different products on the tested areas and should avoid UV and tanning beds exposure.

### Application, Parameter measurement and Instruments

The subjects applied the composition of the present invention and the placebo at the roots of lashes once a day, in the evening, for 60 days. Thereby, they applied the composition on one eye and the placebo on the second eye. The side of application (left or right) of the two treatments was randomized among the volunteers. The composition and the placebo were given to the subjects in anonymous containers which did not provide any information about the treatment - each sample was labelled "right" or "left", indicating the side of application.

The measurement of the parameters was made through image analysis of digital photos of eyes taken before treatment (T₀) and after 15 (T_{15days}), 30 (T_{30days}), and 60 (T_{60days}) days of treatment in a temperature and humidity-controlled room at 24 ± 2 °C and 50 ± 10 % rh.

The parameter measurement was carried out using Fotofinder Dermoscope Ver. 2.0, which is acknowledged as a powerful and versatile system that allows carrying out and memorizing recorded images of any skin surface. The system comprises a high-definition color video-camera with a series of magnifying lenses and a software. The digital images are shown on the screen in their real colors. This allows the observer to examine even the smallest details. Several images can be compared. The same standard floodlight illumination and distance holder are used to take all the images. The software can measure lengths and surfaces on the images like lines, plane curves, circular surfaces, rectangles and polygons. The placement of the camera and of the subject is standardized by using the device 'Head Support'.

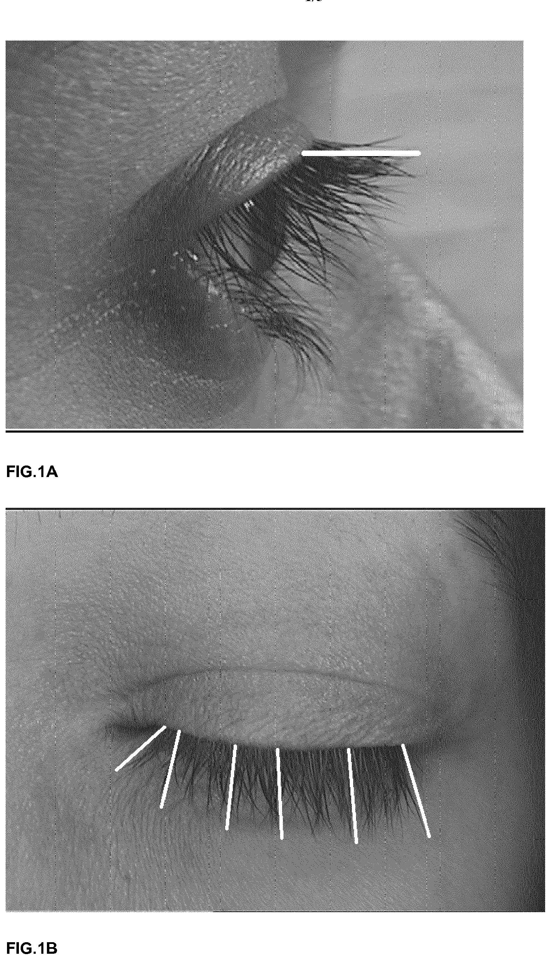

The parameter of maximum length of the eyelashes was determined in the study as the length of a linear segment from the eyelash insertion on the upper eyelid and the point of maximum length of the eyelashes. An exemplary determination of the maximum length of the eyelashes is shown in the photograph of Fig. 1A indicated by the line.

The parameter of average length of the eyelashes was determined in the study as the mean length of 5 linear segments from the eyelash insertion on the upper eyelid and the point of maximum length of 5 eyelashes. An exemplary determination of the average length of the eyelashes is shown in the photograph of Fig. 1B indicated by the lines.

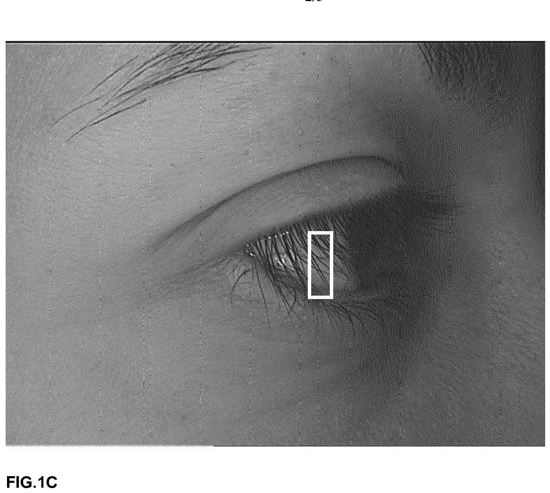

The parameter of eyelashes density was determined in the study as the number of eyelashes in a small section. An exemplary determination of the average length of the eyelashes is shown in the photograph of Fig. 1C indicated by the boxed small section.

### Method of evaluation and statistic analysis

The efficacy of the product is shown by an increase in the parameters, i.e. the maximum and average eyelashes length and the number of eyelashes. Mean values, standard deviations and variations were calculated for each set of values. Following the results of normality test (Kolmogorov-Smirnov test) the instrumental data (T₀, T_{15days}, T_{30days}, T_{60days}) were statistically compared by means of ANOVA for repeated measures and Bonferroni Test for parametric and dependent data, while the variations were statistically compared by means of t-test for parametric and dependent data groups.

The groups of data were considered statistically significant for a probability value p<0.05.

### Results

### 1. Eye lashes maximum length

Table 5 shows the results of the determination of eyelashes maximum length as mean values with standard deviations of the respective test group.

**Table 5**

| | **eyelashes maximum length in mm** | | | |
|---|---|---|---|---|
| | **T₀** | **T_{15days}** | **T_{30days} T_{60days}** | |
| composition | mean 5.09 | mean 5.21 | mean 5.31 | mean 5.50 |
| | std. dev. 0.61 | std. dev. 0.55 | std. dev. 0.64 | std. dev. 0.61 |
| placebo | mean 5.17 | mean 5.15 | mean 5.18 | mean 5.17 |
| | std. dev. 0.58 | std. dev. 0.55 | std. dev. 0.54 | std. dev. 0.57 |

Table 6 shows the statistical evaluation for the increase of eyelashes maximum length: the values depicted in table 6 are the absolute variation and the percentage increase in mm and the respective p-level.

**Table 6**

| | **Composition** | **placebo** |
|---|---|---|
| T_{15days} - T₀ | +0.12 / (+2.4%) / p>0.05 | -0.02 / (-0.4) / p>0.05 |
| T_{30days} - T₀ | +0.22 / (+4.3%) / p<0.001 | +0.01 / (+0.2) / p>0.05 |
| T_{60days} - T₀ | +0.41 / (+8.1%) / p<0.0001 | 0 / (0%) / p>0.05 |

It can be immediately taken from the above that a statistically significant increase in the eyelashes maximum length was detected after 30 and 60 days of active treatment with the composition. An average of +8.1% increase of eyelashes lengthening could be determined at the end of the test. By contrast, no significant variation was found for the placebo treatment.

The increase in the eyelashes maximum length was compared between active treatment with the composition and placebo treatment using a t-test. The results are shown in the following table 7.

**Table 7**

| Table 7: Comparison between treatment with composition and placebo treatment (t-test). | T_{15days} - T₀ | T_{30days} - T₀ | T_{60days} - T₀ |
|---|---|---|---|
| Composition vs. placebo treatment | p<0.01 | p<0.001 | p<0.0001 |

As may be immediately taken from Table 7 above, statistically significant differences between the treatment with the active composition and the placebo treatment were evidenced after 15, 30 and 60 days.

### 2. Average Length

Table 8 shows the results of the determination of eyelashes average length as mean values with standard deviations of the respective test group.

**Table 8**

| | **eye lashes average length in mm** | | | |
|---|---|---|---|---|
| | To | T_{15days} | T_{30days} T_{60days} | |
| composition | mean 5.33std. | mean 5.56std. | mean 5.75 | mean 5.88 |
| | dev.0.70 | dev.0.80 | std. dev. 0.72 | std. dev.0.90 |
| placebo | mean 5.39std. | mean 5.38std. dev. | mean 5.36 | mean 5.35 std. |
| | dev.0.79 | 0.72 | std. dev. 0.80 | dev. 0.75 |

Table 9 shows the statistical evaluation for the increase of eyelashes average length: the values depicted in table 9 are the absolute variation and the percentage increase in mm and the respective p-level.

**Table 9**

| | **Composition** | **placebo** |
|---|---|---|
| T_{15days} - T₀ | +0.23 / (+4.3%) / p>0.05 | -0.01 / (-0.1%) / p>0.05 |
| T_{30days} - T₀ | +0.42 / (+7.9%) / p<0.0001 | -0.03 / (-0.6%) / p>0.05 |
| T_{60days} - T₀ | +0.55 / (+10.3%) / p<0.0001 | -0.04 / (-0.7%) / p>0.05 |

It can be immediately taken from the above that a statistically significant increase in the eyelashes average length was detected after 30 and 60 days of active treatment with the composition. An average increase of eyelashes average lengthening of +10.3% could be determined at the end of the test. By contrast, no significant variation was found for the placebo treatment.

The increase in the eyelashes average length was compared between active treatment with the composition and placebo treatment using a t-test. The results are shown in the following table 10.

**Table 10**

| | T_{15days} - T₀ | T_{30days} - T₀ | T_{60days} - T₀ |
|---|---|---|---|
| Composition vs. placebo treatment | p<0.01 | p<0.001 | p<0.001 |

As may be immediately taken from Table 10 above, statistically significant differences between the treatment with the active composition and the placebo treatment were evidenced after 15, 30 and 60 days.

### 3. Eyelashes Density

Table 11 shows the results of the determination of eyelashes density as mean values with standard deviations of the respective test group.

**Table 11**

| | **eye lashes density** | | | |
|---|---|---|---|---|
| | T₀ | T_{15days} | T_{30days} | T_{60days} |
| composition | mean 10.1 | mean 10.6 | mean 11.1 | mean 11.3 |
| | std. dev. 1.6 | std. dev. 1.7 | std. dev. 1.5 | std. dev. 1.6 |
| placebo | mean 9.6 | mean 9.6 | mean 9.6 | mean 9.5 |
| | std. dev. 1.8 | std. dev. 1.8 | std. dev. 2.0 | std. dev. 1.6 |

Table 12 shows the statistical evaluation for the increase of eyelashes density: the values depicted in table 12 are the absolute variation and the percentage increase in total numbers of eye lashes in the counted segment and the respective p-level.

**Table 12**

| | **Composition placebo** | |
|---|---|---|
| T_{15days} - T₀ | +0.5 / (+5.0%) / p=0.054 | 0 / (0%) / p>0.05 |
| T_{30days}-T₀ | +1.0 / (+9.9%) / p<0.0001 | 0 / (0%) / p>0.05 |
| T_{60days} - T₀ | +1.2 / (+11.9%) / p<0.0001 | -0.1 / (-1.0%) / p>0.05 |

It can be immediately taken from the above that a statistically significant increase in the eyelashes density was detected after 30 and 60 days of active treatment with the composition. After 15 days the increase was near to the statistical significance (p=0.054). An average increase of +11.9% in the number of eyelashes could be determined at the end of the test. By contrast, no significant variation was found for the placebo treatment.

The increase in the eyelashes density was compared between active treatment with the composition and placebo treatment using a t-test. The results are shown in the following table 13.

**Table 13**

| | T_{15days} - T₀ | T_{30days} - T₀ | T_{60days} - T₀ |
|---|---|---|---|
| Composition vs. placebo treatment | p<0.05 | p<0.001 | p<0.0001 |

As may be immediately taken from Table 13 above, statistically significant differences between the treatment with the active composition and the placebo treatment were evidenced after 15, 30 and 60 days.

### Example 5

The composition prepared in Example 1 was administered to a group of horses suffering from hair loss.

Particularly, a first horse subject suffering from hair loss due to abrasion at the tail was subjected to the treatment with the composition.

A second horse subject suffering from hair loss due to a bite wound in the area of the tenderloin was subjected to the treatment with the composition.

Results were compared by photographs, which were taken before the treatment and after 15 days of consecutive treatment. There results were evaluated using eye inspection.

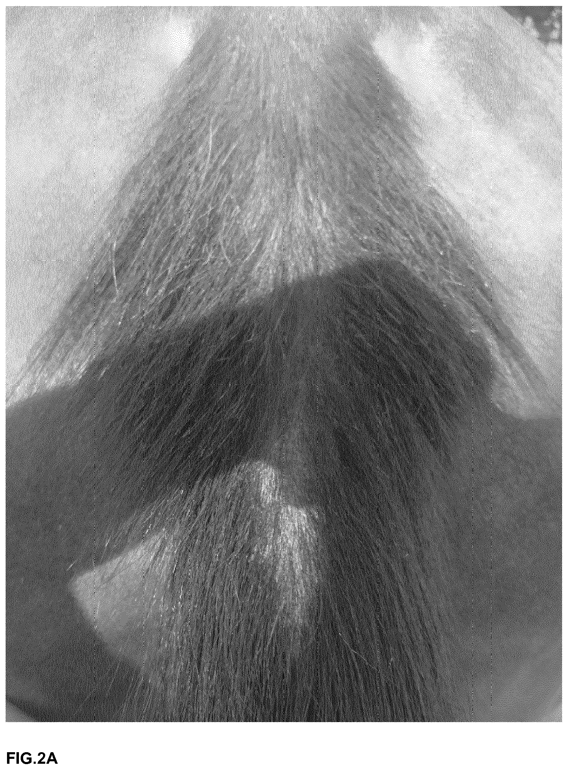

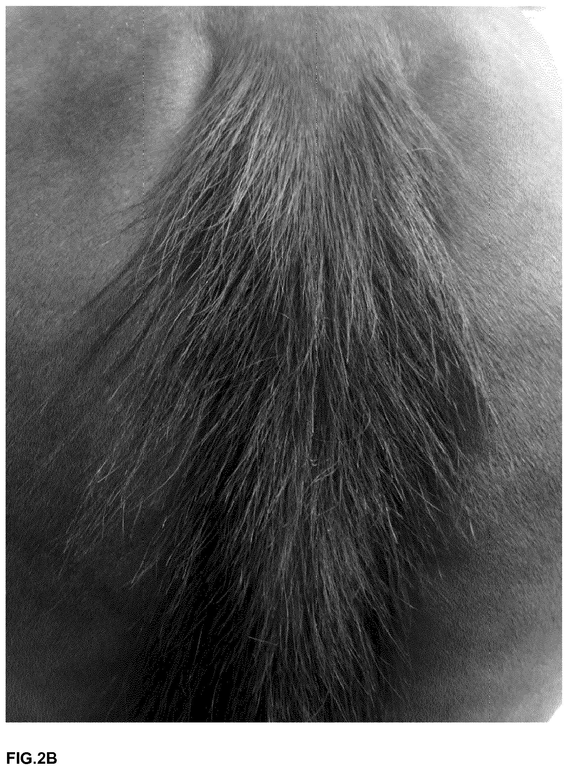

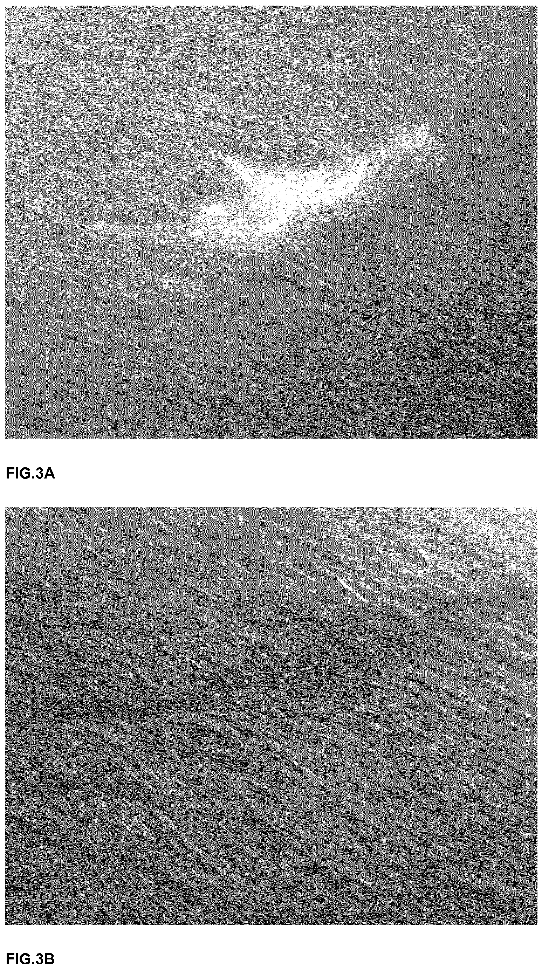

The results for the horse treated at the root of the tail are shown in Figures 2A and 2B, and the results for the horse treated in the area of the tenderloin are shown in Figures 3A and 3B. Thereby, Figures 2A and 3A show the respective horse before the treatment, and Figures 2B and 3B show the respective horse after 15 days of consecutive treatment with the composition of the present invention.

As immediately apparent from the Figures, already after 15 days of consecutive treatment a remarkable increase in length and density of the tail hairs could be observed for the horse subject suffering from hair loss at the tail. And a remarkable increase in length and density of the fur could be observed at the treated area for the second horse subject suffering from hair loss in the area of the tenderloin.

## Claims

1. Active combination comprising
- a botanical extract of *Vigna radiata,*
- a botanical extract of the flower of *Trifolium pratense,*
- a peptide comprising the amino acid sequence Gly-His-Lys from N-terminal to C-terminal end,
wherein the *V. radiata* extract is an extract of the sprout of the mung bean.

2. The active combination of claim 1, comprising a botanical extract of *T*. *pratense* wherein the *T. pratense* extract is an extract of the sprout of the red clover.

3. The active combination of at least one of the preceding claims, wherein the extracts are aqueous or organic solvent extracts.

4. The active combination of at least one of the preceding claims, comprising a peptide which comprises the amino acid sequence Lys-Gly-His-Lys from N-terminal to C-terminal end.

5. The active combination of at least one of the preceding claims, wherein the amount of sprout extracts exceeds the amount of flower extracts on a weight basis.

6. The active combination of at least one of the preceding claims, wherein the amount of *T. pratense* sprout extract exceeds the amount of *T. pratense* flower extract on a weight basis.

7. The active combination of at least one of the preceding claims, wherein the amount of *V*. *radiata* sprout extract exceeds the amount of *T. pratense* flower extract on a weight basis.

8. A cosmetic composition comprising the active combination of at least one of the preceding claims, further comprising water in an amount of at least 50% by weight.

9. The cosmetic composition of claim 8, further comprising at least one preservative with skin-conditioning properties or a mixture of preservatives with skin-conditioning properties in an amount of from 2 to 20% by weight.

10. The cosmetic composition of claim 8 or 9, wherein the composition does not comprise any animal products.

11. A cosmetic method for enhancing eyebrow and/or eyelash growth in a subject, the method comprising the steps of
- administering to the subject an effective amount of the cosmetic composition of one of claims 8 to 10.

12. The method according to claim 11, wherein the composition is administered to the subject by application of the composition to the subject's eyelids and/or eyebrows.

13. The method according to claim 11 or 12, wherein the composition is administered to the subject at least once a day.

14. The method according to one of claims 11 to 13, wherein the composition is administered to the subject at least once a day for a treatment period of at least 3 days.

## Patentansprüche

1. Wirkstoffkombination umfassend
- einen Pflanzenextrakt von *Vigna radiata,*
- einen Pflanzenextrakt von der Blüte von *Trifolium pratense,*
- ein Peptid umfassend die Aminosäuresequenz Gly-His-Lys vom N-terminalen zum C-terminalen Ende,
wobei der *V. radiata*-Extrakt ein Extrakt vom Keimling der Mungbohne ist.

2. Wirkstoffkombination nach Anspruch 1, umfassend einen Pflanzenextrakt von *T. pratense,* wobei der *T. pratense*-Extrakt ein Extrakt vom Keimling des Rotklees ist.

3. Wirkstoffkombination nach mindestens einem der vorhergehenden Ansprüche, wobei die Extrakte wässrige oder organische Lösungsmittelextrakte sind.

4. Wirkstoffkombination nach mindestens einem der vorhergehenden Ansprüche, umfassend ein Peptid, das die Aminosäuresequenz Lys-Gly-His-Lys vom N-terminalen zum C-terminalen Ende umfasst.

5. Wirkstoffkombination nach mindestens einem der vorhergehenden Ansprüche, wobei die Menge an Keimlingextrakten die Menge an Blütenextrakten auf einer Gewichtsbasis übersteigt.

6. Wirkstoffkombination nach mindestens einem der vorhergehenden Ansprüche, wobei die Menge an *T. pratense*-Keimlingextrakt die Menge an *T. pratense*-Blütenextrakt auf einer Gewichtsbasis übersteigt.

7. Wirkstoffkombination nach mindestens einem der vorhergehenden Ansprüche, wobei die Menge an *V. radiata*-Keimlingextrakt die Menge an *T. pratense*-Blütenextrakt auf einer Gewichtsbasis übersteigt.

8. Kosmetische Zusammensetzung umfassend die Wirkstoffkombination nach mindestens einem der vorhergehenden Ansprüche, ferner umfassend Wasser in einer Menge von mindestens 50 Gew.-%.

9. Kosmetische Zusammensetzung nach Anspruch 8, ferner umfassend mindestens ein Konservierungsmittel mit hautpflegenden Eigenschaften oder ein Gemisch von Konservierungsmitteln mit hautpflegenden Eigenschaften in einer Menge von 2 bis 20 Gew.-%.

10. Kosmetische Zusammensetzung nach Anspruch 8 oder 9, wobei die Zusammensetzung keine Tierprodukte umfasst.

11. Kosmetisches Verfahren zur Förderung von Augenbrauen- und/oder Augenwimpernwachstum bei einem Probanden, wobei das Verfahren die folgenden Schritte umfasst
- Verabreichen einer wirksamen Menge der kosmetischen Zusammensetzung nach einem der Ansprüche 8 bis 10 an den Probanden.

12. Verfahren gemäß Anspruch 11, wobei die Zusammensetzung an den Probanden durch Aufbringen der Zusammensetzung auf die Augenlider und/oder Augenbrauen des Probanden verabreicht wird.

13. Verfahren gemäß Anspruch 11 oder 12, wobei die Zusammensetzung an den Probanden mindestens einmal pro Tag verabreicht wird.

14. Verfahren gemäß einem der Ansprüche 11 bis 13, wobei die Zusammensetzung an den Probanden mindestens einmal pro Tag für einen Behandlungszeitraum von mindestens 3 Tagen verabreicht wird.

## Revendications

1. Combinaison d'actifs comprenant
- un extrait botanique de *Vigna radiata,*
- un extrait botanique de fleur de *Trifolium pratense,*
- un peptide comprenant la séquence d'acides aminés Gly-His-Lys de son extrémité N-terminale à son extrémité C-terminale,
dans laquelle l'extrait de *V. radiata* est un extrait de pousse de haricot mungo.

2. Combinaison d'actifs selon la revendication 1, comprenant un extrait botanique de *T. pratense,* dans laquelle l'extrait de *T. pratense* est un extrait de pousse de trèfle rouge.

3. Combinaison d'actifs selon au moins une des revendications précédentes, dans laquelle les extraits sont des extraits de solvants aqueux ou organiques.

4. Combinaison d'actifs selon au moins une des revendications précédentes, comprenant un peptide qui comprend la séquence d'acides aminés Lys-Gly-His-Lys de son extrémité N-terminale à son extrémité C-terminale

5. Combinaison d'actifs selon au moins une des revendications précédentes, dans laquelle la quantité d'extraits de pousses dépasse en poids la quantité d'extraits de fleurs.

6. Combinaison d'actifs selon au moins une des revendications précédentes, dans laquelle la quantité d'extrait de pousses de *T. pratense* dépasse en poids la quantité d'extrait de fleurs de *T. pratense.*

7. Combinaison d'actifs selon au moins une des revendications précédentes, dans laquelle la quantité d'extrait de pousses de *V. radiata* dépasse en poids la quantité d'extrait de fleurs de *T. pratense.*

8. Composition cosmétique comprenant une combinaison d'actifs selon au moins une des revendications précédentes, comprenant en outre au moins 50 % en poids d'eau.

9. Composition cosmétique selon la revendication 8, comprenant en outre entre 2 et 20 % en poids d'au moins un conservateur présentant des propriétés de soin de la peau ou d'un mélange de conservateurs présentant des propriétés de soin de la peau.

10. Composition cosmétique selon la revendication 8 ou 9, dans laquelle la composition ne comprend aucun produit animal.

11. Procédé cosmétique pour favoriser la pousse des sourcils et/ou des cils chez un sujet, ce procédé comprenant les étapes :
- administrer au sujet une quantité efficace de la composition cosmétique selon l'une des revendications 8 à 10.

12. Procédé selon la revendication 11, dans lequel la composition est administrée au sujet par application de la composition sur les paupières et/ou les sourcils du sujet.

13. Procédé selon la revendication 11 ou 12, dans lequel la composition est administrée au sujet au moins une fois par jour.

14. Procédé selon l'une des revendications 11 à 13, dans lequel la composition est administrée au sujet au moins une fois par jour pendant une période de traitement d'au moins 3 jours.
